# EUROPÄISCHE PATENTSCHRIFT

(11) **EP 0 433 835 B1**
(45) Veröffentlichungstag und Bekanntmachung des Hinweises auf die Patenterteilung: **18.05.1994**
(21) Anmeldenummer: 90123722.2
(22) Anmeldetag: 10.12.1990
(51) Int. Cl.: C07D 405/04, C09K 19/34

(54) **Dioxanylpyridine**
Dioxanylpyridines
Dioxanylpyridines

(30) Priorität: 22.12.1989 CH 4623/89
(43) Veröffentlichungstag der Anmeldung: 26.06.1991
(73) Patentinhaber: F. HOFFMANN-LA ROCHE AG, 4002 Basel (CH)
(72) Erfinder: Schadt, Martin, Dr., CH-4411 Seltisberg (CH); Villiger, Alois, Dr., CH-4055 Basel (CH)
(74) Vertreter: Cottong, Norbert A.

(56) Entgegenhaltungen:
- EP-A- 0 391 203
- GB-A- 2 161 481
- US-A- 4 774 020

## Beschreibung

Die vorliegende Erfindung betrifft neue Dioxanylpyridine, deren Herstellung, flüssigkristalline Gemische, die diese Verbindungen enthalten, sowie die Verwendung für elektro-optische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Beispiele solcher Vorrichtungen sind Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("super-birefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannung und einen hohen Kontrast ergeben. Weiterhin sollten sie bei üblichen Betriebstemperaturen von etwa -30°C bis etwa +80°C, insbesondere von etwa -20°C bis etwa +60°C eine geeignete Mesophase besitzen, beispielsweise für die oben genannten Zellen eine nematische oder cholesterische Mesophase. Weitere Eigenschaften, wie die elektrische Leitfähigkeit, die dielektrische Anisotropie und die optische Anisotropie, müssen je nach Zellentyp und Anwendungsbereich unterschiedlichen Anforderungen genügen. Beispielsweise sollten Materialien für Zellen mit verdrillt nematischer Struktur eine positive dielektrische Anisotropie und eine möglichst geringe elektrische Leitfähigkeit aufweisen. Verbindungen, die sich für obengenannte Anwendungen eignen, sind zum Beispiel in EP-A-0 391 293, GB-A-2 161 481 und US-A-4 774 020 beschrieben. Seit einigen Jahren besteht ein besonderes Interesse an aktiv adressierten Flüssigkristallanzeigen, z.B. TFT-Anwendungen ("thin film transistor" = Dünnfilmtransistor) in Fernsehgeräten. Die Verwendung von Cyano-Verbindungen mit positiver dielektrischer Anisotropie führt jedoch in solchen Anzeigen meist zu einem unerwünscht hohen Stromanstieg.

Da Flüssigkristalle in der Regel als Mischungen mehrerer Komponenten zur Anwendung gelangen, ist es wichtig, dass die Komponenten untereinander gut mischbar sind.

Gegenstand der Erfindung sind die Verbindungen der allgemeinen Formel 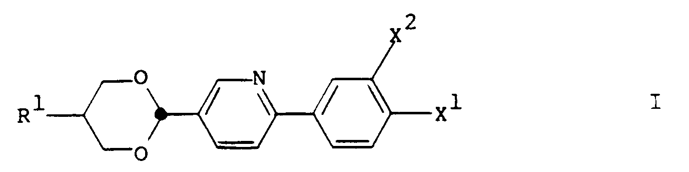
worin X¹ Fluor oder Chlor darstellt; X² Wasserstoff, Fluor oder Chlor bezeichnet; und R¹ eine Alkylgruppe mit höchstens 12 Kohlenstoffatomen bedeutet, in welcher gegebenenfalls eine Gruppe >CH-CH< durch >C=C< ersetzt ist.

Die erfindungsgemässen Verbindungen sind Flüssigkristalle mit hohen Klärpunkten. Zudem werden hochgeordnete smektische Phasen ganz oder weitgehend unterdrückt. Sie weisen überraschend niedrige Viskositäten, insbesondere niedrige Rotationsviskositäten auf und sind gut mischbar mit üblichen Flüssigkristallmaterialien. Trotz der relativ schwachen permanenten Dipolmomente besitzen sie erstaunlich grosse positive dielektrische Anisotropien. Sie sind diesbezüglich zum Teil sogar vergleichbar mit bicyclischen Cyano-Verbindungen, welche aber niedrigere Klärpunkte und höhere Viskositäten aufweisen und bei TFT-Anwendungen zu Leitfähigkeitsproblemen führen. Die erfindungsgemässen Verbindungen ermöglichen daher tiefe Schwellenspannungen und trotzdem kurze Schaltzeiten.

Die erfindungsgemässen Verbindungen eignen sich insbesondere als Komponenten nematischer und cholesterischer Gemische und können dank ihrer guten Löslichkeit untereinander und in bekannten Flüssigkristallen auch in vergleichsweise hohen Konzentrationen verwendet werden.

Die Eigenschaften können je nach Bedeutung der Gruppen R¹, X¹ und X² bis zu einem gewissen Grad variiert werden. Difluor-Derivate (X¹=X²=F) ergeben besonders grosse dielektrische Anisotropien und vergleichsweise kurze Schaltzeiten. p-Chlorderivate (X¹ = Cl) ergeben in der Regel höhere Klärpunkte, und ähnlich kurze Schaltzeiten. 4-Alkenyl führt in der Regel zu tieferen Schwellenspannungen und tieferen k₃₃/k₁₁ und 1E-Alkenyl und 3E-Alkenyl führen in der Regel zu kürzeren Ansprechzeiten, höheren k₃₃/k₁₁ und höheren Klärpunkten im Vergleich zu Alkylresten R¹.

Der Ausdruck "Alkylgruppen, in welcher gegebenenfalls eine Gruppe >CH-CH< durch >C=C< ersetzt ist," umfasst geradkettige und verzweigte, gegebenenfalls chirale Reste, insbesondere Alkyl, 1E-Alkenyl, 3E-Alkenyl, 4-Alkenyl und dergleichen, wie Methyl, Aethyl, Propyl, Butyl, Pentyl, Hexyl, Heptyl, Octyl, Nonyl, Decyl, Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4-Hexenyl, 4-Heptenyl. Geradkettige Reste sind im allgemeinen bevorzugt.

Formel I umfasst die Verbindungen der allgemeinen Formeln 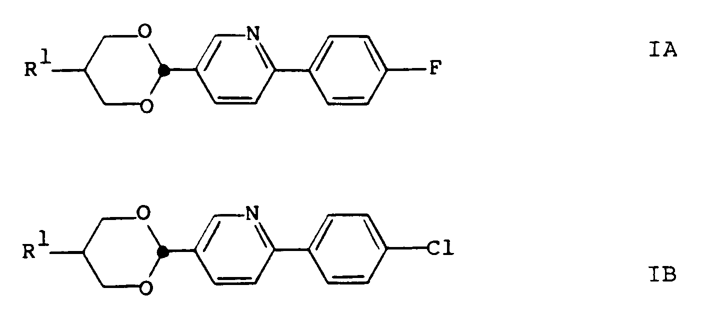 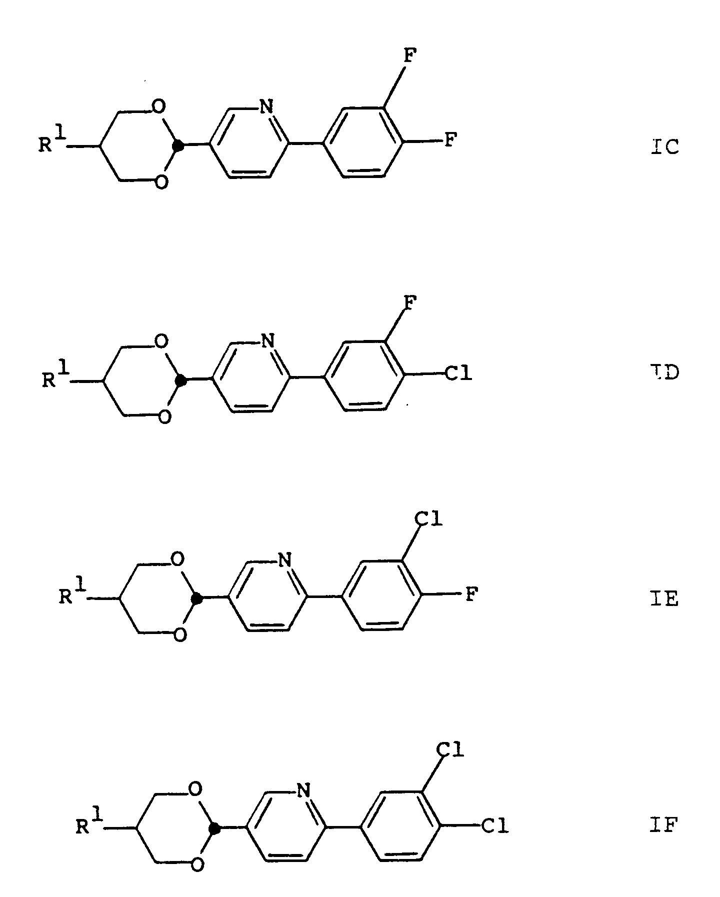
worin R¹ die obige Bedeutung hat.

Vorzugsweise besitzt R¹ höchstens 12 Kohlenstoffatome. Besonders bevorzugte Reste R¹ sind diejenigen mit bis zu 7 Kohlenstoffatomen, insbesondere diejenigen mit bis zu 5 Kohlenstoffatomen.

Vorzugsweise bedeutet R¹ Alkyl, 1E-Alkenyl, 3E-Alkenyl oder 4-Alkenyl, insbesondere C₁-C₁₂-Alkyl, C₂-C₁₂-1E-Alkenyl, C₄-C₁₂-3E-Alkenyl oder C₅-C₁₂-4-Alkenyl.

Die Verbindungen der Formel I können erfindungsgemäss dadurch hergestellt werden, dass man einen Aldehyd der allgemeinen Formel 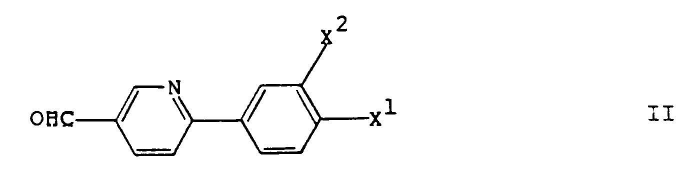
oder ein Acetal hiervon mit einer Verbindung der allgemeinen Formel 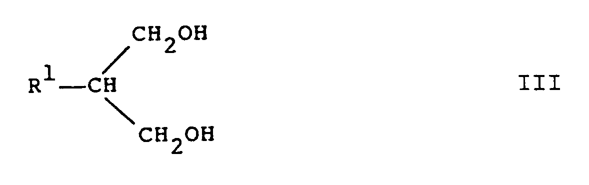
worin R¹, X¹ und X² die obigen Bedeutungen haben,
umsetzt.

Die Umsetzung des Aldehyds der Formel II oder eines geeigneten Acetals (z.B. Dimethylacetal oder Aethylenacetal) mit dem Diol der Formel III kann in an sich bekannter Weise erfolgen. Vorzugsweise erfolgt die Umsetzung in einem inerten organischen Lösungsmittel (beispielsweise einem aromatischen Kohlenwasserstoff, wie Benzol, Toluol oder Xylol) in Gegenwart einer katalytischen Menge einer organischen oder anorganischen Säure, wie p-Toluolsulfonsäure, Schwefelsäure oder trockenem Chlorwasserstoff. Temperatur und Druck sind nicht kritisch. Vorzugsweise wird jedoch bei Atmosphärendruck und Rückflusstemperatur unter Abtrennung des gebildeten Wassers gearbeitet.

Die Ausgangsmaterialien der Formeln II und III sind bekannte oder Analoge bekannter Verbindungen und können nach bekannten Methoden hergestellt werden.

Die Verbindungen der Formel I können in Form von Gemischen untereinander und/oder mit anderen Flüssigkristallkomponenten verwendet werden, wie z.B. mit Substanzen aus den Klassen der Schiffschen Basen, Azobenzole, Azoxybenzole, Phenylbenzoate, Cyclohexancarbonsäurephenylester, Cyclohexancarbonsäurecyclohexylester, Biphenyle, Phenylcyclohexane, Cyclohexylcyclohexane, Phenylpyrimidine, Cyclohexylpyrimidine, Phenyldioxane, 2-Cyclohexyl-1-phenyläthane, Terphenyle, Cyclohexylbiphenyle, Cyclohexylphenylpyrimidine und dergleichen. Derartige Substanzen sind dem Fachmann bekannt und viele davon sind zudem im Handel erhältlich.

Die erfindungsgemässen flüssigkristallinen Gemische enthalten mindestens zwei Komponenten, wovon mindestens eine Komponente eine Verbindung der Formel I ist. Eine zweite und gegebenenfalls weitere Komponenten können weitere Verbindungen der Formel I oder andere Flüssigkristallkomponenten sein. Die Verbindungen der Formel I eignen sich insbesondere für nematische oder, sofern mindestens eine Komponente des Gemisches optisch aktiv ist, auch für cholesterische Gemische.

Aufgrund der guten Löslichkeit der Verbindungen der Formel I und aufgrund ihrer guten Mischbarkeit untereinander kann ihr Anteil in den erfindungsgemässen Gemischen relativ hoch sein. Im allgemeinen ist jedoch ein Anteil von etwa 1-50 Gew.-%, insbesondere etwa 5-30 Gew.-% an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der allgemeinen Formel 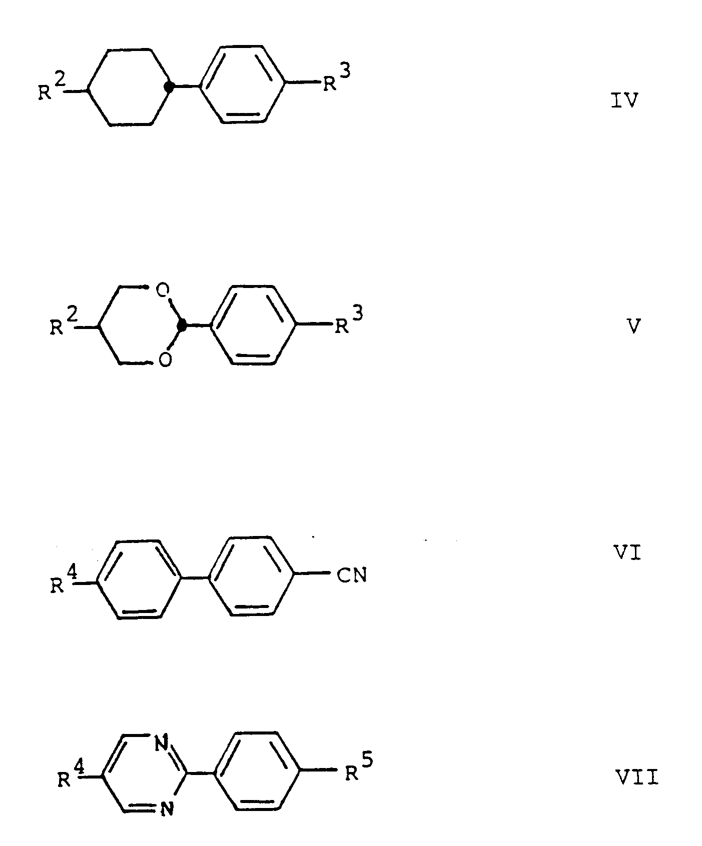 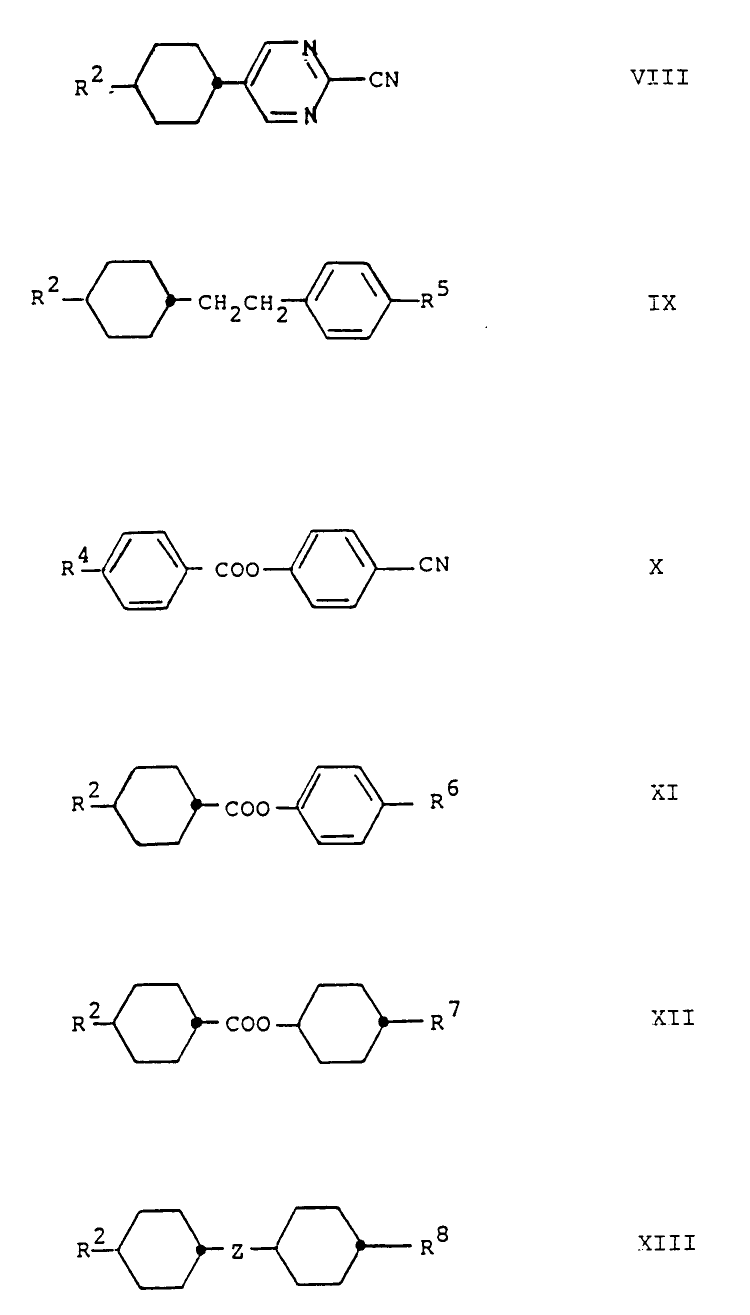 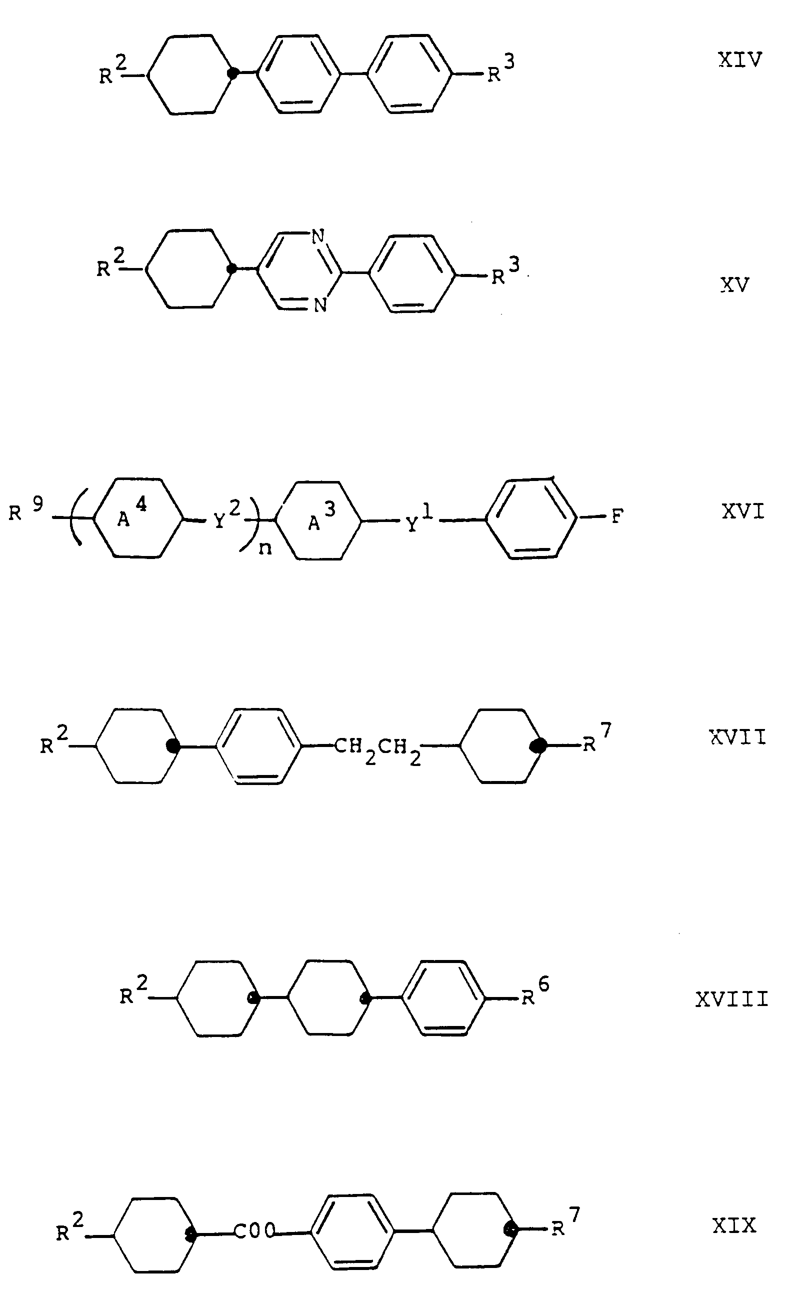 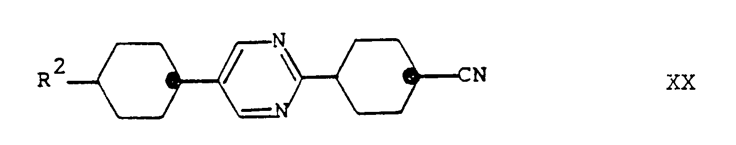
worin R² und R⁷ Alkyl, 1E-Alkenyl, 3E-Alkenyl oder 4-Alkenyl bedeuten; R³ Cyano, -NCS, Alkyl, 1E-Alkenyl, 3E-Alkenyl, 4-Alkenyl, Alkoxy, 2E-Alkenyloxy oder 3-Alkenyloxy bedeutet; R⁴ Alkyl, 3E-Alkenyl oder 4-Alkenyl bedeutet; R⁵ Cyano, -NCS, Alkyl, 3E-Alkenyl, 4-Alkenyl, Alkoxy, 2E-Alkenyloxy oder 3-Alkenyloxy bedeutet; R⁶ Cyano, Alkyl, 3E-Alkenyl, 4-Alkenyl, Alkoxy, 2E-Alkenyloxy oder 3-Alkenyloxy bedeutet; Z eine einfache Kovalenzbindung oder -CH₂CH₂- bedeutet; R⁸ Cyano, Alkoxy, 2E-Alkenyloxy, 3-Alkenyloxy, Alkyl, 1E-Alkenyl, 3E-Alkenyl, 4-Alkenyl, Alkoxymethyl oder 2-Alkenyloxymethyl bedeutet; n für die Zahl 0 oder 1 steht; eine der Gruppen Y¹ und Y² eine einfache Kovalenzbindung, -COO-, -OOC-, -CH₂CH₂-, -CH₂O- oder -OCH₂-und die andere der Gruppen Y¹ und Y² eine einfache Kovalenzbindung bedeutet; die Ringe A³ und A⁴ unabhängig voneinander substituiertes oder unsubstituiertes trans-1,4-Cyclohexylen, in welchem gegebenenfalls 2 nicht benachbarte CH₂-Gruppen durch Sauerstoff ersetzt sind, oder substituiertes oder unsubstituiertes 1,4-Phenylen, in welchem gegebenenfalls 1 oder 2 CH-Gruppen durch Stickstoff ersetzt sind, darstellen; R⁹ Alkyl, 1E-Alkenyl oder, wenn eine der Gruppen Y¹ und Y² eine einfache Kovalenzbindung, -COO-, -OOC- oder -CH₂CH₂-und die andere der Gruppen Y¹ und Y² eine einfache Kovalenzbindung bedeutet, auch 3E-Alkenyl bezeichnet.

Die Alkyl-, Alkenyl-, Alkoxy- und Alkenyloxyreste R²-R⁹ in den Formeln IV-XX sind vorzugsweise geradkettige Reste. Sie weisen vorzugsweise bis zu 12, besonders bevorzugt bis zu 7 Kohlenstoffatome auf.

Die erfindungsgemässen Mischungen können ferner optisch aktive Verbindungen (z.B. optisch aktive 4'-Alkyl- oder 4'-Alkoxy-4-biphenylcarbonitrile) und/oder dichroitische Farbstoffe (z.B. Azo-, Azoxy- oder Anthrachinonfarbstoffe) enthalten. Der Anteil solcher Verbindungen wird durch die Löslichkeit, die gewünschte Ganghöhe, Farbe, Extinktion und dergleichen bestimmt. Im allgemeinen beträgt der Anteil optisch aktiver Verbindungen und dichroitischer Farbstoffe höchstens je etwa 10 Gew.-% im Gesamtgemisch.

Die Herstellung der erfindungsgemässen Gemische und die Herstellung der elektro-optischen Vorrichtungen können in an sich bekannter Weise erfolgen.

Die Herstellung der Verbindungen der Formel I sowie flüssigkristalline Gemische enthaltend diese Verbindungen werden durch die folgenden Bespiele weiter veranschaulicht. C bedeutet eine kristalline, S_{A} eine smektisch A, S_{B} eine smektisch B, N eine nematische und I eine isotrope Phase. V₁₀ und V₅₀ bezeichnen die Spannung für 10% bzw. 50% Transmission, tₒₙ und t_{off} bezeichnen die Einschaltzeit bzw. die Ausschaltzeit und Δn bezeichnet die optische Anisotropie. k₁₁ und k₃₃ bedeuten die elastischen Konstanten für Spreizung bzw. Biegung. Δε bezeichnet die dielektrische Anisotriopie, η die Bulk-Viskosität und γ₁ die Rotationsviskosität. Die elektro-optischen Eigenschaften wurden bei 22°C oder bei einer Temperatur 10°C unterhalb des Klärpunktes gemessen. Sofern nichts angegeben ist, erfolgte die Messung bei 22°C.

### Beispiel 1

a) Ein Gemisch aus 44,3 g 6-Chlor-3-pyridincarbinol, 300 ml Dioxan und 104,3 g 3,4-Dihydro-2H-pyran wurde mit 3 g p-Toluolsulfonsäure-Monohydrat versetzt und 2 Stunden bei 55°C gerührt. Das Reaktionsgemisch wurde mit 10 ml Diäthylamin versetzt und nach dem Erkalten in Diäthyläther aufgenommen. Mehrmaliges Waschen mit Wasser, Trocknen über Natriumsulfat und Einengen im Vakuum lieferte 87,3 g rohes 2-Chlor-5-[(tetrahydro-2-pyranyloxy)methyl]pyridin.
b) Eine aus 1,53 g Magnesium, 10,5 g 1-Brom-4-fluorbenzol und 70 ml Tetrahydrofuran bereitete Grignard-Reagens-Lösung wurde bei 3-7°C innert 45 Minuten in ein Gemisch aus 16,9 g rohem 2-Chlor-5-[tetrahydro-2-pyranyloxy)methyl]pyridin, 60 ml Tetrahydrofuran und 0,62 g 1,3-Bis(diphenylphosphino)propannickel-(II)-chlorid getropft. Das schwarze Reaktionsgemisch wurde noch 1 Std. bei 3°C und 2 Std. bei Raumtemperatur gerührt, und dann mit 200 ml Diäthyläther und 120 ml 0,5 N Ammoniumchlorid-Lösung ausgeschüttelt. Die organische Phase wurde mit Wasser neutral gewaschen, über Natriumsulfat getrocknet, filtriert und eingeengt. Chromatographische Reiningung des Rückstands an 230 g Kieselgel mit Hexan/Aethylacetat (Vol. 9:1) lieferte 13,4 g 2-(4-Fluorphenyl)-5-[(tetrahydro-2-pyranyloxy)methyl]pyridin als gelbliches Oel.
c) Eine Lösung von 13,4 g 2-(4-Fluorphenyl)-5-[(tetrahydro-2-pyranyloxy)methyl]pyridin in 250 ml Tetrahydrofuran wurde mit 45 ml 2N Salzsäure versetzt. Das Gemisch wurde über Nacht bei 60°C gerührt, dann mit 100 ml Diäthyläther verdünnt, einmal mit 50 ml gesättigter Natriumcarbonat-Lösung und zweimal mit je 60 ml Wasser gewaschen, über Natriumsulfat getrocknet und eingeengt. Es resultierten 8,1 g rohes 6-(4-Fluorphenyl)-3-pyridincarbinol.
d) Eine Lösung von 8,1 g 6-(4-Fluorphenyl)-3-pyridincarbinol in 200 ml Aethylenchlorid wurde mit 13,1 g aktiviertem Mangandioxid 7 Stunden gekocht. Die Suspension wurde dann filtriert. Einengen des Filtrats und chromatographische Reinigung des Rückstandes an 150 g Kieselgel mit Hexan/Aethylacetat (Vol. 4:1) ergab 6,2 g 6-(4-Fluorphenyl)-3-pyridincarboxaldehyd als bräunlichen Festkörper.
e) Eine Lösung von 1,51 g 6-(4-Fluorphenyl)-3-pyridincarboxaldehyd und 1,10 g 2-Propyl-1,3-propandiol in 50 ml Toluol wurde mit 10 Tropfen 10-prozentiger (v/v) Schwefelsäure versetzt. Das Gemisch wurde 1,5 Stunden zum Sieden erhitzt, wobei feuchtes Toluol abdestilliert und durch frisches Toluol ersetzt wurde. Dann wurde das Reaktionsgemisch mit Triäthylamin neutralisiert und nach dem Erkalten dreimal mit Wasser gewaschen, über Natriumsulfat getrocknet und eingeengt. Chromatographische Reinigung des Rückstandes an 60 g Kieselgel mit Toluol/Aceton (Vol. 99:1) und zweimalige Umkristallisation des trans/cis-Gemisches aus Hexan/Aethylacetat (Vol. 1:1) ergab 0,51 g reines 5-(trans-5-Propyl-1,3-dioxan-2-yl)-2-(4-fluorphenyl)pyridin; Smp. (C-S_{A}) 106,5°C, S_{A}-N 117,5°C, Klp. (N-I) 163°C.

In analoger Weise können folgende Verbindungen hergestellt werden:
5-(trans-5-Aethyl-1,3-dioxan-2-yl)-2-(4-fluorphenyl)pyridin;
5-(trans-5-Butyl-1,3-dioxan-2-yl)-2-(4-fluorphenyl)pyridin, Smp. (C-S_{A}) 86,0°C, S_{A}-N 138,5°C, Klp. (N-I) 156°C;
5-(trans-5-Pentyl-1,3-dioxan-2-yl)-2-(4-fluorphenyl)pyridin;
5-(trans-5-Heptyl-1,3-dioxan-2-yl)-2-(4-fluorphenyl)pyridin;
5-(trans-5-Vinyl-1,3-dioxan-2-yl)-2-(4-fluorphenyl)pyridin;
5-[trans-5-(1E-Propenyl)-1,3-dioxan-2-yl]-2-(4-fluorphenyl)pyridin, Smp. (C-N) 138,4°C, Klp. (N-I) 202°C;
5-[trans-5-(1E-Butenyl)-1,3-dioxan-2-yl]-2-(4-fluorphenyl)pyridin;
5-[trans-5-(1E-Pentenyl)-1,3-dioxan-2-yl]-2-(4-fluorphenyl)pyridin;
5-[trans-5-(3-Butenyl)-1,3-dioxan-2-yl]-2-(4-fluorphenyl)pyridin;
5-[trans-5-(4-Pentenyl)-1,3-dioxan-2-yl]-2-(4-fluorphenyl)pyridin;
5-(trans-5-Aethyl-1,3-dioxan-2-yl)-2-(3,4-difluorphenyl)pyridin;
5-(trans-5-Propyl-1,3-dioxan-2-yl)-2-(3,4-difluorphenyl)pyridin, Smp. (C-S_{A}) 100,4°C, S_{A}-N 117,5°C, Klp. (N-I) 125°C;
5-(trans-5-Butyl-1,3-dioxan-2-yl)-2-(3,4-difluorphenyl)pyridin;
5-(trans-5-Pentyl-1,3-dioxan-2-yl)-2-(3,4-difluorphenyl)pyridin;
5-(trans-5-Heptyl-1,3-dioxan-2-yl)-2-(3,4-difluorphenyl)pyridin;
5-(trans-5-Vinyl-1,3-dioxan-2-yl)-2-(3,4-difluorphenyl)pyridin;
5-[trans-5-(1E-Propenyl)-1,3-dioxan-2-yl]-2-(3,4-difluorphenyl)pyridin, Smp. (C-S_{A}) 121°C, S_{A}-N 137°C, Klp. (N-I) 159,5°C;
5-[trans-5-(1E-Butenyl)-1,3-dioxan-2-yl]-2-(3,4-difluorphenyl)pyridin;
5-[trans-5-(1E-Pentenyl)-1,3-dioxan-2-yl]-2-(3,4-difluorphenyl)pyridin;
5-[trans-5-(3-Butenyl)-1,3-dioxan-2-yl]-2-(3,4-difluorphenyl)pyridin;
5-[trans-5-(4-Pentenyl)-1,3-dioxan-2-yl]-2-(3,4-difluorphenyl)pyridin;
5-(trans-5-Aethyl-1,3-dioxan-2-yl)-2-(4-chlorphenyl)pyridin;
5-(trans-5-Propyl-1,3-dioxan-2-yl)-2-(4-chlorphenyl)pyridin;
5-(trans-5-Butyl-1,3-dioxan-2-yl)-2-(4-chlorphenyl)pyridin;
5-(trans-5-Pentyl-1,3-dioxan-2-yl)-2-(4-chlorphenyl)pyridin;
5-(trans-5-Heptyl-1,3-dioxan-2-yl)-2-(4-chlorphenyl)pyridin;
5-(trans-5-Vinyl-1,3-dioxan-2-yl)-2-(4-chlorphenyl)pyridin;
5-[trans-5-(1E-Propenyl)-1,3-dioxan-2-yl]-2-(4-chlorphenyl)pyridin;
5-[trans-5-(1E-Butenyl)-1,3-dioxan-2-yl]-2-(4-chlorphenyl)pyridin;
5-[trans-5-(1E-Pentenyl)-1,3-dioxan-2-yl]-2-(4-chlorphenyl)pyridin;
5-[trans-5-(3-Butenyl)-1,3-dioxan-2-yl]-2-(4-chlorphenyl)pyridin;
5-[trans-5-(4-Pentenyl)-1,3-dioxan-2-yl]-2-(4-chlorphenyl)pyridin;
5-(trans-5-Propyl-1,3-dioxan-2-yl)-2-(4-chlor-3-fluorphenyl)pyridin;
5-(trans-5-Butyl-1,3-dioxan-2-yl)-2-(4-chlor-3-fluorphenyl)pyridin;
5-(trans-5-Pentyl-1,3-dioxan-2-yl)-2-(4-chlor-3-fluorphenyl)pyridin;
5-[trans-5-(1E-Propenyl)-1,3-dioxan-2-yl]-2-(4-chlor-3-fluorphenyl)pyridin;
5-[trans-5-(1E-Butenyl)-1,3-dioxan-2-yl]-2-(4-chlor-3-fluorphenyl)pyridin;
5-[trans-5-(1E-Pentenyl)-1,3-dioxan-2-yl]-2-(4-chlor-3-fluorphenyl)pyridin;
5-[trans-5-(3-Butenyl)-1,3-dioxan-2-yl]-2-(4-chlor-3-fluorphenyl)pyridin;
5-[trans-5-(4-Pentenyl)-1,3-dioxan-2-yl]-2-(4-chlor-3-fluorphenyl)pyridin;
5-(trans-5-Propyl-1,3-dioxan-2-yl)-2-(3,4-dichlorphenyl)pyridin;
5-(trans-5-Butyl-1,3-dioxan-2-yl)-2-(3,4-dichlorphenyl)pyridin;
5-[trans-5-(1E-Propenyl)-1,3-dioxan-2-yl]-2-(3,4-dichlorphenyl)pyridin.

### Beispiel 2

Zur Untersuchung der Eigenschaften der Verbindungen der Formel I in Gemischen wurden binäre Mischungen mit 4-(trans-4-Pentylcyclohexyl)benzonitril hergestellt. Die Schwellenspannung und die Ansprechzeiten wurden in einer TN-Zelle (low bias tilt) mit 8 µm Plattenabstand gemessen; als Betriebsspannung wurde der 2,5-fache Wert der Schwellenspannung gewählt. Die entsprechenden Daten für reines 4-(trans-4-Pentylcyclohexyl)benzontril betragen: Klp. (N-I) 54,6°C, V₁₀ = 1,62 V, tₒₙ = 30 ms, t_{off} = 42 ms, Δn = 0,120.

### Mischung A

90 Gew.-% 4-(trans-4-Pentylcyclohexyl)benzonitril,
10 Gew.-% 5-(trans-5-Propyl-1,3-dioxan-2-yl)-2-(4-fluorphenyl)pyridin;
Klp. (N-I) 59,4°C, V₁₀ = 1,38 V, tₒₙ = 27 ms, t_{off} = 43 ms, Δn = 0,125.

### Mischung B

80 Gew.-% 4-(trans-4-Pentylcyclohexyl)benzonitril,
20 Gew.-% 5-(trans-5-Propyl-1,3-dioxan-2-yl)-2-(4-fluorphenyl)pyridin;
Klp. (N-I) 66,4°C, V₁₀ = 1,38 V, tₒₙ = 29 ms, t_{off} = 48 ms, Δn = 0,128.

### Mischung C

90 Gew.-% 4-(trans-4-Pentylcyclohexyl)benzonitril,
10 Gew.-% 5-(trans-5-Propyl-1,3-dioxan-2-yl)-2-(3,4-difluorphenyl)pyridin;
Klp. (N-I) 56,4°C, V₁₀ = 1,33 V, tₒₙ = 28 ms, t_{off} = 45 ms, Δn = 0,122.

### Mischung D

80 Gew.-% 4-(trans-Pentylcyclohexyl)benzonitril,
20 Gew.-% 5-(trans-5-Propyl-1,3-dioxan-2-yl)-2-(3,4-difluorphenyl)pyridin;
Klp. (N-I) 58,8°C, V₁₀ = 1,23 V, tₒₙ = 31 ms, t_{off} = 55 ms, Δn = 0,121.

### Mischung E

90 Gew.-% 4-(trans-4-Pentylcyclohexyl)benzonitril,
10 Gew.-% 5-[trans-5-(1E-Propenyl)-1,3-dioxan-2-yl]-2-(4-fluorphenyl)pyridin;
Klp. (N-I) 60,6°C, V₁₀ = 1,41 V, tₒₙ = 27 ms, t_{off} = 45 ms, Δn = 0,126.

### Mischung F

80 Gew.-% 4-(trans-4-Pentylcyclohexyl)benzonitril,
20 Gew.-% 5-[trans-5-(1E-Propenyl)-1,3-dioxan-2-yl]-2-(4-fluorphenyl)pyridin;
Klp. (N-I) 68,8°C, V₁₀ = 1,41 V, tₒₙ = 29 ms, t_{off} = 47 ms, Δn = 0,124.

### Mischung G

90 Gew.-% 4-(trans-4-Pentylcyclohexyl)benzonitril,
10 Gew.-% 5-[trans-5-(1E-Propenyl)-1,3-dioxan-2-yl]-2-(3,4-difluorphenyl)pyridin;
Klp. (N-I) 56,7°C, V₁₀ = 1,34 V, tₒₙ = 29 ms, t_{off} = 47 ms, Δn = 0,125,

### Mischung H

80 Gew.-% 4-(trans-4-Pentylcyclohexyl)benzonitril,
20 Gew.-% 5-[trans-5-(1E-Propenyl)-1,3-dioxan-2-yl]-2-(3,4-difluorphenyl)pyridin;
Klp. (N-I) 60,8°C, V₁₀ = 1,24 V, tₒₙ = 34 ms, t_{off} = 53 ms, Δn = 0,128.

## Patentansprüche

1. Verbindungen der allgemeinen Formel 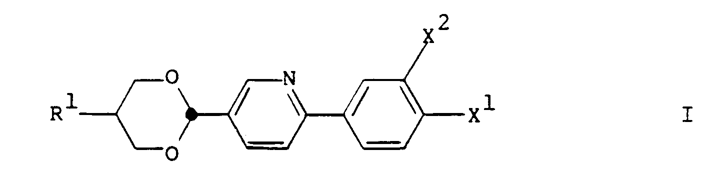 worin X¹ Fluor oder Chlor darstellt; X² Wasserstoff, Fluor oder Chlor bezeichnet; und R¹ eine Alkylgruppe mit höchstens 12 Kohlenstoffatomen bedeutet, in welcher gegebenenfalls eine Gruppe >CH-CH< durch >C=C< ersetzt ist.

2. Verbindungen nach Anspruch 1, dadurch gekennzeichnet, dass R¹ einen geradkettigen Rest bedeutet.

3. Verbindungen nach Anspruch 1 oder 2, dadurch gekennzeichnet, dass R¹ höchstens 12, vorzugsweise höchstens 7 Kohlenstoffatome aufweist.

4. Verbindungen nach einem der Ansprüche 1 bis 3, dadurch gekennzeichnet, dass R¹ Alkyl, 1E-Alkenyl, 3E-Alkenyl oder 4-Alkenyl bedeutet.

5. Flüssigkristallines Gemisch mit mindestens zwei Komponenten, dadurch gekennzeichnet, dass mindestens eine Komponente eine Verbindung der in Anspruch 1 definierten Formel I ist.

6. Flüssigkristallines Gemisch nach Anspruch 5, dadurch gekennzeichnet, dass der Anteil an Verbindungen der Formel I 1-50, vorzugsweise 5-30 Gew.-% beträgt.

7. Verfahren zur Herstellung der Verbindungen der in Anspruch 1 definierten Formel I, dadurch gekennzeichnet, dass man einen Aldehyd der allgemeinen Formel 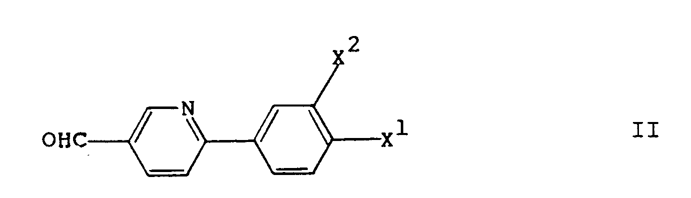 oder ein Acetal hiervon mit einer Verbindung der allgemeinen Formel 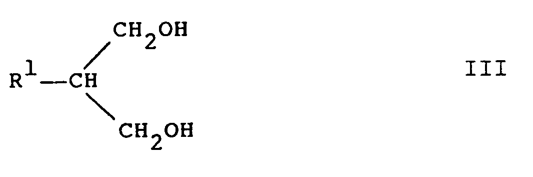 worin R¹, X¹ und X² die in Anspruch 1 gegebenen Bedeutungen haben,
umsetzt.

8. Verwendung der Verbindungen der in Anspruch 1 definierten Formel I für elektro-optische Zwecke.

## Claims

1. Compounds of the general formula 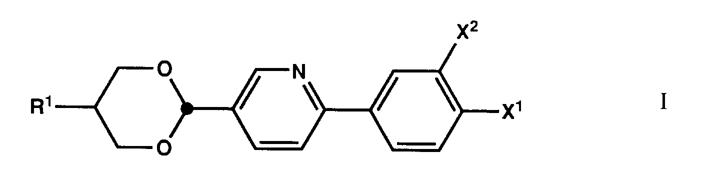 wherein X¹ represents fluorine or chlorine; X² denotes hydrogen, fluorine or chlorine; and R¹ signifies an alkyl group with a maximum of 12 carbon atoms in which optionally a >CH-CH< group is replaced by >C=C<.

2. Compounds according to claim 1, characterized in that R¹ signifies a straight-chain residue.

3. Compounds according to claim 1 or 2, characterized in that R¹ has a maximum of 12, preferably a maximum of 7, carbon atoms.

4. Compounds according to any one of claims 1 to 3, characterized in that R¹ signifies alkyl, 1E-alkenyl, 3E-alkenyl or 4-alkenyl.

5. A liquid crystalline mixture having at least two components, characterized in that at least one component is a compound of formula I defined in claim 1.

6. A liquid crystalline mixture according to claim 5, characterized in that the content of compounds of formula I is 1-50, preferably 5-30, wt.%.

7. A process for the manufacture of the compounds of formula I defined in claim 1, characterized by reacting an aldehyde of the general formula 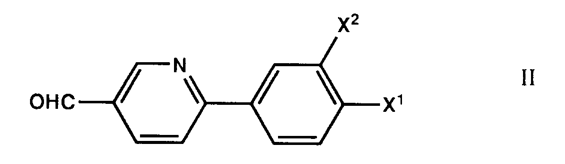 or an acetal thereof with a compound of the general formula 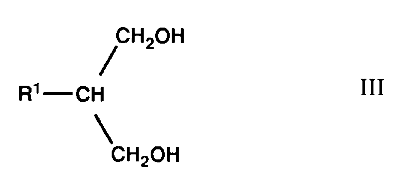 wherein R¹, X¹ and X² have the significances given in claim 1.

8. The use of the compounds of formula I defined in claim 1 for electro-optical purposes.

## Revendications

1. Composés de formule générale 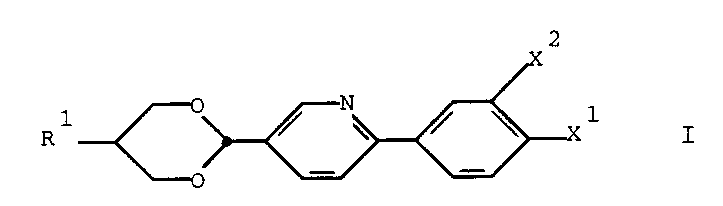 dans laquelle X¹ représente le fluor ou le chlore ; X² représente l'hydrogène, le fluor ou le chlore ; et R¹ représente un groupe alkyle à 12 atomes de carbone au maximum et dans lequel le cas échéant un groupe >CH-CH< est remplacé par >C=C<.

2. Composés selon la revendication 1, caractérisés en ce que R¹ est un groupe à chaîne droite.

3. Composés selon la revendication 1 ou 2, caractérisés en ce que R¹ contient au maximum 12 et de préférence au maximum 7 atomes de carbone.

4. Composés selon l'une des revendications 1 à 3, caractérisés en ce que R¹ représente un groupe alkyle, 1E-alcényle, 3E-alcényle ou 4-alcényle.

5. Mélange à cristaux liquides à au moins deux composants, caractérisé en ce qu'il contient au moins un composant consistant en un composé de formule I de la revendication 1.

6. Mélange à cristaux liquides selon la revendication 5, caractérisé en ce que la proportion des composés de formule I est de 1 à 50, de préférence de 5 à 30 % en poids.

7. Procédé de préparation des composés de formule I de la revendication 1, caractérisé en ce que l'on fait réagir un aldéhyde de formule générale : 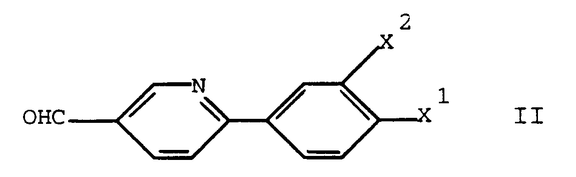 ou l'un de ses acétals avec un composé de formule générale : 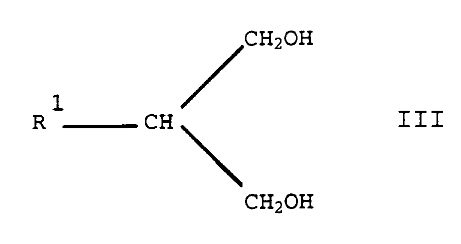 R¹, X¹ et X² ayant les significations indiquées dans la revendication 1.

8. Utilisation des composés de formule I de la revendication 1 dans des applications électro-optiques.
